# FASCICULE DE BREVET EUROPEEN

(11) **EP 0 235 480 B1**
(45) Date de publication et mention de la délivrance du brevet: **10.03.1993**
(21) Numéro de dépôt: 86402911.1
(22) Date de dépôt: 23.12.1986
(51) Int. Cl.: G01N 25/14, B01D 11/02

(54) **Procedé et appareillage de caracterisation de la matière organique extractible par au moins un solvant dans des conditions de temperature et de pression supercritiques**
Verfahren und Vorrichtung zur Charakterisierung eines mit mindestens einem Lösungsmittel unter überkritischen Druck- und Temperaturbedingungen extrahierbaren organischen Materials
Method and apparatus for the characterization of organic material extractable by at least one solvent under conditions of supercritical temperature and pressure

(30) Priorité: 30.12.1985 FR 8519463
(43) Date de publication de la demande: 09.09.1987
(73) Titulaire: INSTITUT FRANCAIS DU PETROLE, 92502 Rueil-Malmaison (FR)
(72) Inventeur: Durand, Bernard, F-92500 Rueil Malmaison (FR); Espitalie, Michel, F-92500 Rueil Malmaison (FR); Monin, Jean-Claude, F-95240 Cormeilles en Parisis (FR); Perrut, Michel, f-54210 Saint Nicolas de Port (FR); Parmentier, François, F-69007 Lyon (FR)

(56) Documents cités:
- GB-A- 2 104 132
- US-A- 3 923 847
- CHEMICAL ABSTRACTS, vol. 98, no. 24, 13 juin 1983, page 157, résumé no. 201110h, Columbus, Ohio, US; R.D. SMITH et al.: "New method for the direct analysis of supercritical fluid coal extraction and liquefaction",& FUEL 1983, 62(4), 466-8
- CHEMICAL ABSTRACTS, vol. 100, no. 14, 2 avril 1984, page 168, résumé no. 106286w, Columbus, Ohio, US; R.D. SMITH et al.: "Supercritical fluid methods for coal extraction, separation, and analysis", & PREPR. PAP. - AM. CHEM. SOC., DIV. FUEL CHEM. 1983, 28(4), 235-40

## Description

La présente invention concerne un procédé de caractérisation d'un paramètre relatif à de la matière organique extractible par au moins un solvant dans des conditions de température et de pression supercritique et un appareillage pour la mise en oeuvre de ce procédé. Elle s'applique plus particulièrement à la caractérisation de cette matière organique contenue par exemple dans les roches sédimentaires et intéresse à ce titre, par exemple, les domaines pétrolier et charbonnier.

Par matière organique extractible par au moins un solvant, on entend de la matière que l'on peut extraire par exemple d'une roche, de sédiments géologiques anciens ou récents, du charbon, de sables ou schistes bitumineux, d'huiles ou de résidus asphaltiques, et de toute matrice minérale ou organique, naturelle ou synthétique.

Elle peut s'appliquer aussi au domaine des substances naturelles d'origine végétale ou animale.

A titre d'exemple, on s'intéressera en particulier à la matière organique de sédiments géologiques.

Pour caractériser la matière organique, il est connu de soumettre un échantillon de roche à une extraction par un solvant organique, tel que du chloroforme ou du benzène, miscible à l'extrait obtenu, puis d'analyser après séparation partielle ou totale du solvant et de manière indépendante et séparée, l'extrait ainsi obtenu. Cette méthode présente surtout l'inconvénient de faire disparaître, au moins en partie, les composés légers (dont la molécule contient au plus quatorze atomes de carbone) au moment des transferts et de l'évaporation des solvants qui, de ce fait, ne sont plus représentatifs de la quantité réellement présente. D'autre part, la discontinuité du processus en deux étapes nettement différenciées rend la méthode longue et difficilement automatisable.

Il existe par ailleurs, pour caractériser la matière organique extractible, la méthode dite de thermovaporisation qui consiste à désorber vers 300°C les hydrocarbures les plus volatils contenus dans un échantillon de roche et à les analyser en ligne par exemple, par chromatographie en phase gazeuse. Cette méthode thermique permet sensiblement d'obtenir une information caractéristique de la matière organique pouvant être extraite par des solvants. Les hydrocarbures légers sont, dans ce cas, présents et analysés ; en revanche, les hydrocarbures les plus lourds risquent d'être sous évalués dans la mesure où ils peuvent être retenus dans la roche et/ou être dégradés thermiquement avec pour conséquence une production d'hydrocarbures notamment légers qui vont avoir tendance à s'ajouter, lors de la mesure, à ceux déjà présents dans la roche. En raison des niveaux thermiques atteints, la matière organique qui n'est pas directement volatilisable va aussi avoir tendance à se dégrader, ce qui va entraîner la production d'hydrocarbures néoformés qui vont s'ajouter à ceux directement volatilisables et qui vont alors fausser les mesures.

Un premier objet de l'invention est donc de pouvoir analyser sensiblement la quasi totalité de la matière organique extractible qui soit vraiment représentative de l'échantillon avec un minimum de risques d'artefact.

Par ailleurs, dans le cas où l'on utilise une méthode chromatographique pour caractériser l'extrait à la suite d'une extraction par un ou plusieurs solvants organiques miscibles, ce processus présente deux inconvénients majeurs : le premier est lié à l'interaction des produits analysés et du solvant en quantité importante qui peut "aveugler" le système de détection et en tout état de cause rendre quasiment impossible la caractérisation des fractions d'une volatilité voisine de celle du solvant. Ainsi, les méthodes d'analyse les plus performantes font appel à la chromatographie en phase gazeuse utilisant des dætecteurs très sensibles comme ceux à ionisation de flamme ; or, dans ce cas, tous les solvants organiques habituellement utilisés sont très "visibles" en ionisation de flamme et il est impossible d'y parer, ce qui rend la méthode d'analyse par ce type de détection inopérante pour les fractions les plus légères et de point d'ébullition voisin de celui du solvant considéré. L'autre inconvénient est qu'il est nécessaire d'évaporer l'excès de solvant ce qui entraine la perte au moins partielle des molécules légères.

Enfin, l'étude d'un bassin sédimentaire implique la mise à disposition d'un grand nombre d'échantillons qui sont pour la plupart, de très faibles quantités de carottes (de 0,01g à 10g par exemple) et leur analyse doit être rapide, facile à réaliser avec un minimum d'intervention humaine de façon à minimiser les pertes d'hydrocarbures légers par transfert et dans la mesure du possible, se prêter à une automatisation.

L'invention remédie à ces inconvénients et répond au problème posé. Elle concerne plus particulièrement un procédé de caractérisation d'un paramètre relatif à la matière organique dans lequel on extrait un échantillon comprenant de la matière organique, par un mélange comprenant au moins un solvant, l'extraction étant effectuée à une température et à une pression au moins égales à la température critique et à la pression critique du solvant, de façon à produire une solution d'extrait comprenant au moins une molécule de matière organique, on effectue la mesure dudit paramètre à partir de ladite molécule et on produit à partir de cette mesure au moins un signal représentatif dudit paramètre relatif à la matière organique, ledit procédé étant caractérisé en ce qu'on fait circuler le solvant en circuit fermé pendant un temps suffisamment long de façon à obtenir un équilibre entre l'échantillon et la solution d'extrait, ladite mesure étant effectuée sensiblement auxdites température et pression d'extraction.

Cette molécule peut être par exemple un composé organique, tel qu'un hydrocarbure ou un composé comprenant un hétéroatome comme l'oxygène, le soufre et/ou l'azote.

De manière avantageuse, on maintient, selon l'invention, ladite solution sensiblement à la température et à la pression d'extraction lors du prélèvement de l'extrait soumis à la mesure.

En opérant dans les conditions ci-dessus, c'est-à-dire en conditions supercritiques et en circuit fermé, on minimise les condensations d'hydrocarbures lourds.

Par ce procédé, on réalise en ligne et en une seule fois, l'extraction et l'analyse de la matière organique extractible. L'opération est rapide et facile à mettre en oeuvre. L'analyse que l'on obtient dans la mesure où elle est effectuée en ligne, c'est-à-dire sensiblement dans les conditions de l'extraction sans manipulation de l'échantillon, est représentative de l'échantillon extrait avec, notamment, un minimum de pertes d'hydrocarbures légers et également d'hydrocarbures lourds, par suite de la quasi absence d'hydrocarbures néoformés à partir de ces hydrocarbures lourds.

Par ailleurs, le procédé se prête à une automatisation dans la mesure où, de façon avantageuse, on peut opérer sur la base de faibles prélévements d'échantillons qui peuvent varier par exemple de 0,01 à 10 grammes de roche.

Avantageusement, on peut effectuer l'étape d'extraction à une température comprise entre la température critique du solvant et environ 100°C au-dessus de cette température. On a obtenu d'excellents résultats lorsqu'on opérait à une température comprise entre 10°C et 50°C au dessus de la température critique.

La pression selon le procédé peut avantageusement être comprise entre la pression critique du solvant utilisé et 300 bar au-dessus de cette pression. On a obtenu tout particulièrement de bons résultats lorsqu'on opérait sous une pression comprise entre 30 et 150 bar audessus de la pression critique.

Le détecteur adapté à produire le signal qui sera mesuré sera choisi par exemple parmi les détecteurs de chromatographes en phase liquide tels que le détecteur en lumière ultraviolette, le réfractomètre différentiel, le détecteur à indice de viscosité, les détecteurs de chromatographes en phase gazeuse tels que détecteur à ionisation de flamme, à photométrie de flamme, à photoionisation, catharomètre, spectromètre de masse, détecteur en lumière infrarouge... Le choix sera fonction du paramètre étudié ; s'il s'agit par exemple de suivre en continu l'extraction d'au moins une molécule sensible au rayonnement ultraviolet ou même à une longueur d'onde spécifique d'absorption, on pourra adopter un détecteur ultraviolet dont le signal sera intégré en fonction du temps.

De façon avantageuse, pour supporter les températures et pressions voisines ou supérieures aux conditions critiques, on pourra utiliser une cellule à fenêtres en saphir qui présentent une grande résistance mécanique.

Selon une caractéristique de l'invention, il sera avantageux, après ou pendant l'étape d'extraction, de prélever en ligne sensiblement dans les conditions du procédé au moins une partie (une aliquote) de l'extrait sur laquelle on effectue en ligne la mesure du signal.

Cette façon de procéder est particulièrement intéressante quand on désire utiliser des détecteurs très sensibles comme les détecteurs à ionisation de flamme qui ne nécessitent que des quantités infimes de matière. Selon un autre mode de réalisation on peut réaliser en ligne par chromatographie en phase gazeuse, éventuellement en condition supercritique, une analyse détaillée de l'extrait prélevé et injecté en ligne, en séparant grâce à une colonne chromatographique adaptée au type de mélange, capillaire ou remplie, les divers constituants de l'extrait et on détecte ensuite, par exemple en ionisation de flamme, un signal caractéristique d'au moins une molécule des constituants ainsi séparés. Ce signal est ensuite traité suivant des techniques connues. Mais il peut être tout aussi intéressant de substituer ce type de détection par une détection telle que la spectométrie de masse et même d'obtenir par exemple une double détection. Selon un autre mode de réalisation, on peut injecter en ligne une aliquote de l'extrait qui a été prélevé dans un chromatographe liquide haute performance équipée d'une colonne d'adsorption, d'exclusion ou d'échanges d'ions par exemple, adaptée au problème à analyser, en aval de laquelle on va disposer un détecteur tel que décrit dans la description.

Les solvants utilisés peuvent être des solvants organiques. Avantageusement ils comprennent au moins un hydrocarbure ou au moins un hydrocarbure substitué par des halogènes ayant de 3 à 12 atomes de carbone par molécule et de préférence 5 à 10 atomes de carbone. Parmi les hydrocarbures aromatiques préférés, on peut citer le benzène, le toluène, le xylène. Le toluène est tout particulièrement intéressant en raison de sa moindre toxicité.

Des composés hétérocycliques peuvent aussi être utilisés, comme la pyridine, le furanne ou le thiophène et leurs dérivés.

Parmi les solvants aromatiques, les composés hydrocarbonés cycliques ou acycliques sont aussi de bons solvants. On peut citer avantageuavantageusement les cycloalcanes et leurs formes substituées et de préférence le cyclohexane et le méthylcyclohexane.

Les composés hydrocarbonés acycliques comprennent avantageusement des alcanes de 3 à 12 atomes de carbone, qui peuvent être linéaires ou ramifiés. L'hexane et l'heptane sont des solvants préférés. Parmi les hydrocarbures substitués, on peut citer avantageusement le chloroforme, le chlorure de méthylène et le tetrachlorure de carbone.

Tous ces solvants sont avantageusement utilisés à une température ou à une pression supérieure à la température critique : ils sont alors nommés fluides en état supercritique. Dans ces conditions, ils présentent l'avantage de posséder une masse volumique et un pouvoir solvant comparables à ceux d'un liquide courant avec le plus souvent une viscosité plus faible et une diffusivité plus grande, ce qui en fait un solvant séduisant.

Il est possible également d'opérer dans les conditions décrites selon l'invention en présence d'un solvant non organique et avantageusement dans des conditions supercritiques d'utilisation. C'est ainsi qu'on a obtenu d'excellents résultats avec le dioxyde de carbone dont les propriétés critiques ne conduisent pas à des conditions d'utilisation trop difficiles (pression critique : 72,8 bar - température critique : 31,3°C - masse volumique critique : 428 kg/m³), et dans lequel la détection des composés du mélange étudié est facile et sans risque de chevauchement de signaux.

L'invention concerne aussi un dispositif pour la mise en oeuvre du procédé. Plus particulièrement, il comprend en combinaison :
- des moyens pour mettre en circulation en circuit fermé, dans un sens ou dans l'autre, un mélange comprenant au moins un solvant à une pression et à une température au moins égale à la pression critique et à la température critique dudit solvant,
- un organe d'extraction contenant ledit échantillon et placé sur ledit circuit, ledit organe produisant une solution d'extrait qui comprend au moins une molécule dudit extrait, et
- au moins un appareil de mesure produisant un signal représentatif dudit paramètre à partir de ladite molécule dudit extrait, ce paramètre étant mesuré sensiblement auxdites température et pression d'extraction, l'appareil étant relié audit organe d'extraction et auxdits moyens de mise en circulation.

Ces moyens décrits ainsi combinés coopèrent entre eux pour aboutir à un résultat surprenant, à savoir la possibilité d'une analyse représentative de la matière organique extractible présente dans l'échantillon, incluant les hydrocarbures tant légers que lourds.

De manière avantageuse, le dispositif peut comporter au moins un organe de prélévement d'au moins une partie aliquote de l'extrait tel qu'une vanne d'injection à boucle, utilisée de manière connue en chromatographie liquide à haute performance. Cet organe est interposé entre l'organe d'extraction et l'appareil de production et de mesure du signal, et permet de prélever au moins une partie aliquote de l'extrait sensiblement à la température et à la pression selon le procédé de l'invention.

Les moyens de mise en circulation en conditions de température et de pression critiques comportent un organe de pompage sous pression et de régulation de pression relié à l'organe d'extraction, des moyens de chauffage tels qu'une enceinte thermostatée, qui maintiennent l'organe d'extraction, l'organe de prélévement et le circuit fermé à la température et à la pression désirées et définies selon l'invention.

L'invention sera mieux comprise par la description de quelques modes de réalisation, donnés à titre illustratif mais nullement limitatif, qui en sera faite ci-après à l'aide des figures annexées :
- la figure 1 représente un mode de réalisation du procédé selon l'invention,
- la figure 2 montre une vanne d'injection à boucle disposée dans le circuit fermé selon l'invention,
- les figures 3 et 3A illustrent une représentation schématique de la cellule d'extraction et de l'autoclave porte-cellule, et
- la figure 4 montre un chromatogramme obtenu selon l'invention, tandis que les figures 5 et 6 représentent des chromatogrammes réalisés selon l'art antérieur, le premier après une extraction au chloroforme et le second par thermovaporisation.

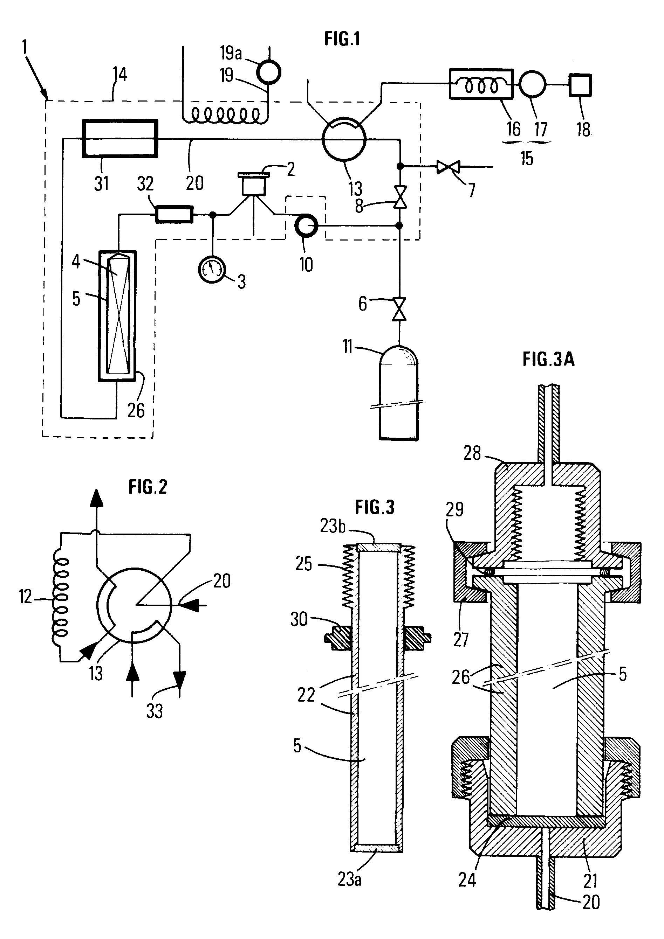

Sur la figure 1, on a représenté le dispositif 1 selon l'invention comprenant un organe ou cellule d'extraction 5 contenant le produit à caractériser 4, produit éventuellement broyé finement afin de faciliter le contact avec le fluide solvant (le dioxyde de carbone par exemple) qui va être utilisé en état supercritique.

Le solvant est envoyé par une ligne en circuit fermé 20 et une pompe à haute pression 10 dans la cellule d'extraction.

Un réservoir 11 et une vanne 6 reliés à la ligne 20 et à la pompe 10 alimentent la cellule d'extraction 5.

La pression désirée est maintenue grâce à un organe de régulation de pression non représenté sur le schéma.

La ligne 20, la cellule d'extraction 5, des organes de sécurité et de mesure de pression tels qu'une soupape de sécurité 2 et un manomètre 3, des vannes de prélèvement 13 et d'isolement 7 à 9 de circuit dont on verra le fonctionnement ci-dessous, sont portées et maintenues à une température selon le procédé par des moyens de chauffage 19 et des moyens de régulation 19**a** disposés dans une enceinte thermostatée 14, par exemple une enceinte 14 à air pulsé ou un bain liquide, permettant de maintenir sensiblement constante la température d'entrée des fluides dans la cellule d'extraction 5.

Un élément de filtrage 32 entre la pompe 10 et l'organe d'extraction stoppe les particules supérieures à 2µm (2 × 10⁻⁶m).

Au moins une vanne de prélèvement 13 a été disposée en ligne sur le circuit, par exemple entre la cellule 5 et la vanne d'isolation 7 du dispositif d'analyse 15 et elle permet à tout moment, soit durant l'extraction, soit après l'extraction, de prélever dans les conditions de l'extraction une partie aliquote de l'échantillon extrait.

Le dispositif de production d'un signal 15, tel qu'un chromatographe en phase gazeuse, est relié à la vanne de prélèvement 13, par exemple à six voies, de type classique que l'on rencontre dans les appareillages en chromatographie liquide haute performance. Il comprend une colonne chromatographique 16 capillaire ou remplie, alimentée par le gaz vecteur (N₂ par exemple) qui balaie par une autre boucle 33 de la vanne 13 la boucle d'injection 12 (Fig. 2) au moment choisi par l'opérateur. Un détecteur à ionisation de flamme 17 analyse ensuite les composés séparés par la colonne chromatographique 16 et le signal émis est intégré de manière connue afin d'obtenir par exemple la proportion des molécules identifiables sur le chromatogramme obtenu caractéristique de l'extrait.

Selon les figures 3 et 3A, l'organe d'extraction 5 comprend un tube métallique 22, par exemple de diamètre 0,6 à 0,7 cm et d'épaisseur 1 à 2 mm à une extrémité duquel se trouve un fritté serti 23**a** de porosité par exemple égale à 2µm (2 × 10⁻⁶m) et à l'autre extrémité duquel se présente un filetage 25. Sur ce filetage va reposer un autre fritté non serti 23**b**. L'échantillon de roche finement broyé (mode sensiblement égal à 50 microns (50 × 10⁻⁶m) par un broyeur par exemple à anneaux est introduit dans la cellule 5 entre les deux frittés puis tassé.

La cellule est ensuite introduite dans un autoclave porte-cellule 26. Un jeu de raccords 21, 28 métalliques, de colliers 27 ainsi que des joints métalliques 29 et en téflon 30 assurent l'étanchéité de l'ensemble tandis qu'un fritté supplémentaire de même porosité que le fritté 23a minimise le risque de voir la roche finement broyée circuler dans le circuit 20.

La procédure de mise en oeuvre du procédé s'effectue de la façon suivante :
- Mise en place du solide 4 dans la cellule d'extraction 5 qui est alors hermétiquement close, laissant la possibilité au fluide d'entrer et de sortir sans entraîner de particules solides grâce à l'utilisation de matériaux filtrants 23**a**, 23**b** et 24 (métal fritté par exemple) ;
- purge de la cellule par le fluide à l'état gazeux par l'ouverture des vannes 6 et 7, la vanne 8 étant fermée : ainsi l'air présent dans le circuit 20 et les vannes de prélèvement 13 est remplacé par le fluide à l'état gazeux qui n'a alors pratiquement aucun pouvoir solvant sur les fractions présentes dans l'échantillon à caractériser ;
- remplissage de la cellule 5 et du circuit 20, après isolement du circuit de l'atmosphère en fermant les vannes 7 et en ouvrant la vanne 6, par pompage du fluide du réservoir 11. Le fonctionnement de la pompe est arrêté quand la pression atteint le niveau souhaité ; on ferme alors la vanne 6 de sortie du réservoir ;
- extraction en circuit fermé après ouverture de la vanne 8 pendant un temps supposé suffisamment long pour que l'équilibre entre l'échantillon et le fluide chargé des fractions extraites dudit échantillon soit supposé réalisé. On arrête alors la pompe 10 ;
- injection du mélange solvant-extrait contenu dans la boucle 12 de la vanne de prélèvement 13 après fermeture de la vanne 7 ;
- analyse classique en chromatographie en phase gazeuse 15, 16, 17, 18 avec programmation de la température éventuelle pour améliorer la séparation des différents constituants ;
- après qu'on ait réalisé l'injection dans l'analyseur 15, le circuit est décomprimé par ouverture de la vanne 7 et la cellule d'extraction 5 peut être ouverte et nettoyée ; on peut alors éventuellement refermer le circuit à vide (sans échantillon) pour réaliser un nettoyage du circuit 20 avec le fluide solvant pompé à haute pression et évacué à la pression atmosphérique à travers la vanne 7.

Comme présenté sur la figure 2, la boucle 12 est insérée dans le circuit principal dans les phases purge, remplissage et extraction alors qu'à ce moment-là le gaz vecteur du chromatographe balaie la boucle 33. Dans la phase injection, on manoeuvre la vanne 13 de façon à isoler le circuit principal de la boucle 12, laquelle est alors balayée par le gaz vecteur vers le chromatographe, réalisant ainsi l'injection du mélange solvant-extrait contenu dans cette boucle. On peut alors manoeuvrer en sens inverse la vanne d'injection 13 provoquant un nouveau remplissage par le fluide solvant maintenu sous pression dans le reste du circuit. Le volume de la boucle d'injection étant en général très faible par rapport au volume du circuit, ces manoeuvres d'injection-remplissage de la boucle peuvent être réalisées plusieurs fois sans provoquer une décompression sensible du circuit 20 qui perturberait l'équilibre solvant-échantillon.

Après avoir réalisé les injections dans l'analyseur 15 en nombre suffisant, on procède au nettoyage des circuits comme précédemment.

Selon une variante du dispositif, il est intéressant de disposer en série sur le circuit fermé, par exemple entre la cellule d'extraction 5 et la vanne de prélèvement 13 dans la direction d'écoulement du mélange, une cellule 31 sensible au rayonnement ultraviolet à fenêtre en saphir par exemple, de façon à suivre par exemple en continu et en fonction du temps l'absorption d'au moins une molécule à une longueur d'onde donnée.

On ne sortira pas du cadre de l'invention en utilisant avec le solvant au moins un désorbant tel que des alcools à faible nombre de carbone comme par exemple le méthanol ou l'éthanol, l'oxyde d'éthylène et l'époxypropane.

De même, par exemple, dans le cas d'extraction d'huiles asphaltiques, on ne sortira pas du cadre de l'invention en adoptant des solvants organiques miscibles de bas poids moléculaire choisis parmi le groupe des naphtas, des essences de craquage catalytique ou de cokéfaction, des huiles de craquage (light cycle oil), l'éthylène glycol et le thiophène en présence éventuellement d'un catalyseur du groupe VI ou VIII.

Après extraction et analyse, le mélange comprenant la solution de solvant et d'extrait peut être séparé, le solvant recyclé, l'extrait soumis à d'autres procédés de traitement.

Les exemples qui suivent servent à illustrer l'invention mais ne peuvent en aucun cas limiter sa portée.

Les chiffres N portés sur les figures indiquent les n-alcanes à N atomes de carbone tandis que les lettres B, T, X représentent le benzène, le toluène et le xylène.

### Exemple 1

On réalise dans une cellule d'extraction de volume total d'environ 1cm³ l'extraction sur la base de faible prélèvement d'un échantillon de sédiment géologique en provenance d'un bassin sédimentaire pétrolifère (Mahakam, Indonésie) finement broyé (50 × 10⁻⁶ µm) de masse m = 40mg en présence de gaz carbonique dans des conditions supercritiques : pression 160 bar, température 60°C. Le débit de CO₂ est de l'ordre de 200g/h et on opère en excès de solvant de façon à être en dessous du seuil de solubilité dans le solvant.

L'extraction est considérée achevée au bout d'un temps t = 20min.

Le prélèvement est effectué en ligne par une vanne à six voies à boucle d'injection telle que décrite ci-dessus et ce prélèvement est envoyé à un débit de 2ml/min de gaz vecteur, en l'occurrence de l'hélium, dans une colonne chromatographique de type capillaire (longueur 25 m, diamètre 0,32 mm) phase stationnaire apolaire : CPSil 5 de Chrompack (marque déposée) et avec un détecteur à ionisation de flamme sensible aux hydrocarbures contenus dans l'extrait. L'injection s'est déroulée à une température de 300°C. La colonne capillaire du chromatographe en phase gazeuse a été chauffée de -60°C à 300°C, à 3°C par minute.

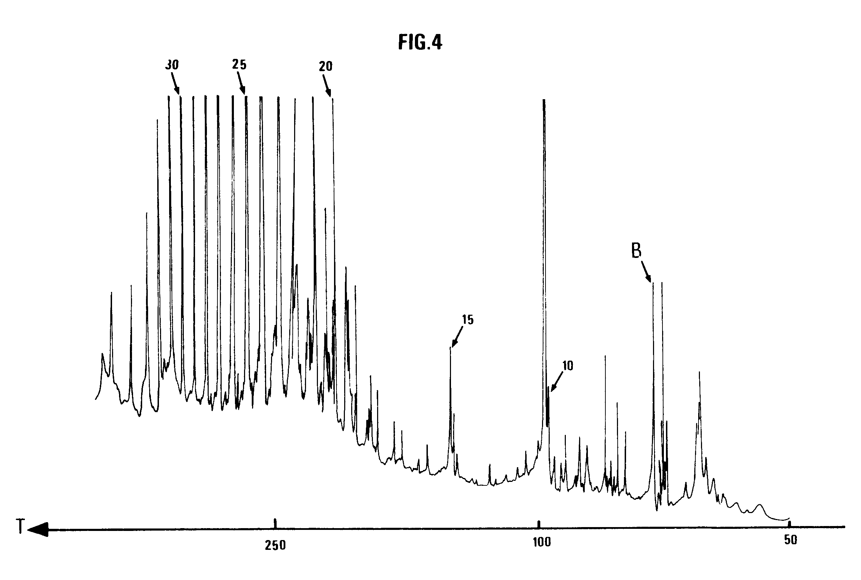

Le chromatogramme obtenu est représenté sur la figure 4.

L'identification des pics est réalisée selon une technique connue et l'intégration de chaque pic peut être également déterminée par une technique connue de l'homme de métier.

On note sur le chromatogramme la présence d'hydrocarbures légers originels et notamment d'hydrocarbures aliphatiques saturés comme les n-alcanes avec 4 à 14 atomes de carbone et également la présence d'hydrocarbures lourds, notamment les n-alcanes ayant de 14 à 32 atomes de carbone.

### Exemple 2 : Selon l'art antérieur

On réalise l'extraction de 5 g du même sédiment broyé dans un bécher contenant 50 ml de chloroforme à une température de 50°C pendant 1 heure. Après extraction, le solvant est évaporé, l'extrait est dissout dans de l'isopentane et envoyé en partie sur une colonne chromatographique identique à celle de l'exemple 1 dans les mêmes conditions de débit. La température de la colonne est programmée de 100 à 300°C à 3°C par minute.

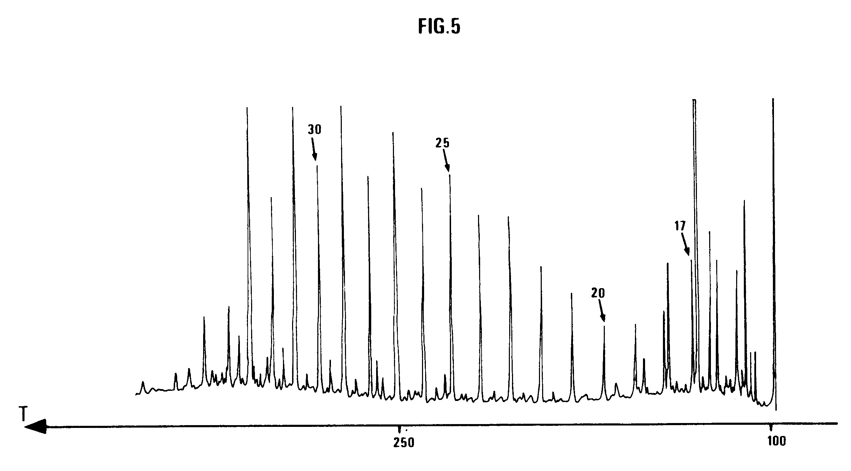

On note sur le chromatogramme illustré par la figure 5 l'absence d'hydrocarbures légers ayant moins de 14 atomes de carbone et une bonne représentativité des hydrocarbures lourds centrès autour du n-alcane à 30 atomes de carbone.

### Exemple 3 : Selon l'art antérieur

On réalise la thermovaporisation de 50 microgrammes du même sédiment broyé que celui de l'exemple 1, en pyrolysant dans un microfour l'échantillon à 50°C/min de la température ambiante à 300°C. Les hydrocarbures sont piégés en tête de colonne chromatographique qui est identique à celle de l'exemple 1, à une température de -50°C. La température de la colonne est ensuite amenée de -50°C à 10°C à +10°C/min et de 10 à 300°C à 3°C/min.

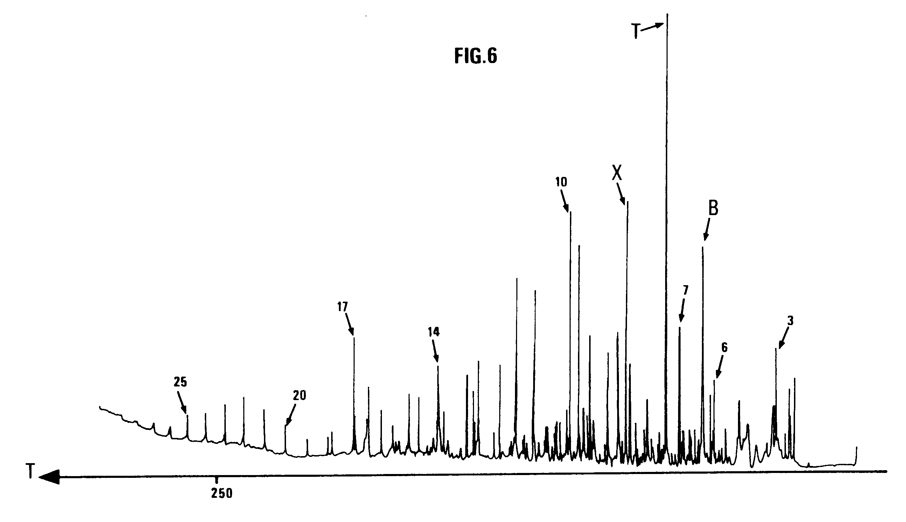

Le chromatogramme illustré par la figure 6 montre la présence d'une quantité plus importante d'hydrocarbures légers originels et néoformés par craquage des hydrocarbures lourds et il montre aussi la présence d'une faible quantité d'hydrocarbures lourds.

## Revendications

1. Procédé de caractérisation d'un paramètre relatif à de la matière organique dans lequel on extrait un échantillon comprenant de la matière organique, par un mélange comprenant au moins un solvant, l'extraction étant effectuée à une température et à une pression au moins égales à la température critique et à la pression critique du solvant, de façon à produire une solution d'extrait comprenant au moins une molécule de matière organique, on effectue la mesure dudit paramètre à partir de ladite molécule et on produit à partir de cette mesure au moins un signal représentatif dudit paramètre relatif à la matière organique, ledit procédé étant caractérisé en ce que l'on fait circuler le solvant en circuit fermé pendant un temps suffisamment long de façon à obtenir un équilibre entre l'échantillon et la solution d'extrait, ladite mesure étant effectuée sensiblement auxdites température et pression d'extraction.

2. Procédé selon la revendication 1, caractérisé en ce qu'on prélève durant l'extraction sensiblement auxdites température et pression d'extraction une partie aliquote dudit extrait à partir de laquelle on produit ledit signal.

3. Procédé selon la revendication 1, caractérisé en ce qu'on prélève, en fin d'extraction, sensiblement auxdites température et pression d'extraction, une partie aliquote dudit extrait à partir de laquelle on produit ledit signal.

4. Procédé selon l'une des revendications 1 à 3, caractérisé en ce que le solvant utilisé est le dioxyde de carbone.

5. Procédé selon l'une des revendications 1 à 3, caractérisé en ce que le solvant utilisé comprend au moins un hydrocarbure ayant de 3 à 12 atomes de carbone.

6. Procédé selon l'une quelconque des revendications 2 à 5, caractérisé en ce qu'on produit ledit signal à partir d'une analyse par chromatographie.

7. Procédé selon la revendication 6, caractérisé en ce qu'on produit ledit signal à partir d'une analyse par chromatographie en phase gazeuse.

8. Procédé selon l'une des revendications 1 à 7, caractérisé en ce qu'on effectue l'étape d'extraction à une température comprise entre la température critique du solvant et une température supérieure d'environ 100°C à ladite température critique.

9. Dispositif pour la mise en oeuvre du procédé selon la revendication 1, caractérisé en ce qu'il comprend en combinaison :
- des moyens (10, 19) pour mettre en circulation en circuit fermé (20) dans un sens ou dans l'autre, un mélange comprenant au moins un solvant à une pression et à une température au moins égale à la pression critique et à la température critique dudit solvant,
- un organe d'extraction (5) contenant ledit échantillon et placé sur ledit circuit, ledit organe produisant une solution d'extrait qui comprend au moins une molécule dudit extrait, et
- au moins un appareil de mesure (15) produisant un signal représentatif dudit paramètre à partir de ladite molécule dudit extrait, ce paramètre étant mesuré sensiblement auxdites température et pression d'extraction, l'appareil (15) étant relié audit organe d'extraction (5) et auxdits moyens de mise en circulation (10).

10. Dispositif selon la revendication 9, caractérisé en ce qu'il comporte, en outre, au moins un organe (13) prélevant auxdites température et pression d'extraction une partie aliquote dudit extrait interposé entre l'organe d'extraction et l'appareil (15) de production et de mesure du signal.

11. Dispositif selon l'une des revendications 9 ou 10, caractérisé en ce que les moyens de mise en circulation comportent un organe de pompage (10) sous pression relié à l'organe d'extraction (15) et des moyens de chauffage (19) dudit organe d'extraction (5), dudit organe de prélèvement (13) et dudit circuit (20).

12. Dispositif selon l'une des revendications 9 à 11, caractérisé en ce que l'appareil de mesure (15) produisant ledit signal comporte un chromatographe comprenant au moins un détecteur (17).

13. Dispositif selon la revendication 12, caractérisé en ce que le chromatographe est un chromatographe en phase gazeuse et le détecteur choisi dans le groupe comprenant le détecteur à ionisation de flamme, le catharomètre, le détecteur à photométrie de flamme, à photoionisation, à lumière infrarouge et le spectromètre de masse.

14. Application du procédé selon l'une des revendications 1 à 8, à l'extraction d'hydrocarbures contenus dans les roches sédimentaires.

## Claims

1. Method of characterising a parameter relating to organic matter from which a sample comprising organic matter is extracted by means of a mixture comprising at least one solvent, the extraction being carried out at a temperature and pressure at least equal to the critical temperature and pressure of the solvent, so as to produce an extract solution comprising at least one molecule of organic matter, the measurement of the said parameter is made from the said molecule and from this measurement at least one signal is produced representing the said parameter relating to the organic matter, the said method being characterised in that the solvent is circulated in closed circuit for a sufficiently long time so as to obtain equilibrium between the sample and the extract solution, the said measurement being made substantially at the said extraction temperature and pressure.

2. Method according to Claim 1, characterised in that, during the extraction substantially at the said extraction temperature and pressure, an aliquot of the said extract is taken from which the said signal is produced.

3. Method according to Claim 1, characterised in that, at the end of the extraction, substantially at the said extraction temperature and pressure, an aliquot of the said extract is taken from which the said signal is produced.

4. Method according to one of Claims 1 to 3, characterised in that the solvent used is carbon dioxide.

5. Method according to one of Claims 1 to 3, characterised in that the solvent used comprises at least one hydrocarbon with 3 to 12 carbon atoms.

6. Method according to any one of Claims 2 to 5, characterised in that the said signal is produced from an analysis by chromatography.

7. Method according to Claim 6, characterised in that the said signal is produced from an analysis by gas chromatography.

8. Method according to one of Claims 1 to 7, characterised in that the extraction stage is carried out at a temperature between the critical temperature of the solvent and a temperature approximately 100°C higher than the said critical temperature.

9. Device for implementing the method according to Claim 1, characterised in that it comprises in combination:
- means (10, 19) for circulating in closed circuit (20), in one direction or the other, a mixture comprising at least one solvent at a pressure and temperature at least equal to the critical pressure and temperature of the said solvent,
- an extraction unit (5) containing the said sample and placed in the said circuit, the said unit producing an extract solution which comprises at least one molecule of the said extract, and
- at least one measuring apparatus (15) producing a signal representing the said parameter from the said molecule of the said extract, this parameter being measured substantially at the said extraction temperature and pressure, the apparatus (15) being connected to the said extraction unit (5) and to the said circulation means (10).

10. Device according to Claim 9, characterised in that it also includes at least one device (13) taking, at the said extraction temperature and pressure, an aliquot of the said extract interposed between the extraction unit and the signal producing and measuring apparatus (15).

11. Device according to one of Claims 9 or 10, characterised in that the circulation means include a pressurised pumping unit (10) connected to the extraction unit (15) and means (19) for heating the said extraction unit (5), the said sampling device (13) and the said circuit (20).

12. Device according to one of Claims 9 to 11, characterised in that the measuring apparatus (15) producing the said signal includes a chromatograph comprising at least one detector (17).

13. Device according to Claim 12, characterised in that the chromatography is a gas chromatography and the detector chosen from the group comprising flame ionisation detectors, catharometers, flame photometry, photoionisation and infrared detectors and mass spectrometers.

14. Application of the method according to one of Claims 1 to 8 to the extraction of hydrocarbons contained in sedimentary rocks.

## Patentansprüche

1. Verfahren zur Charakterisierung eines Parameters bezüglich organischen Materials, bei dem man eine ein organisches Material enthaltende Probe durch ein wenigstens ein Lösungsmittel enthaltendes Gemisch extrahiert, wobei die Extraktion vorgenommen wird bei einer Temperatur und einem Druck, die wenigstens gleich der kritischen Temperatur und dem kritischen Druck des Lösungsmittels derart sind, daß eine Extraktionslösung mit wenigstens einem Molekül organischen Materials erzeugt wird, man die Messung dieses Parameters ausgehend von diesem Molekül vornimmt und ausgehend von dieser Messung wenigstens ein für diesen Parameter, bezogen auf das organische Material, repräsentatives Signal erzeugt, **dadurch gekennzeichnet,** daß man das Lösungsmittel in geschlossenem Kreislauf während eines ausreichend langen Zeitraums zirkulieren läßt, derart, daß man ein Gleichgewicht zwischen der Probe und der Extraktionslösung erhält, wobei die Messung im wesentlichen bei Extraktionstemperatur und -druck vorgenommen wird.

2. Verfahren nach Anspruch 1, dadurch gekennzeichnet, daß man während der Extraktion im wesentlichen bei dieser Extraktionstemperatur und diesem Extraktionsdruck einen aliquoten Teil dieses Extrakts entnimmt, ausgehend von dem man dieses Signal erzeugt.

3. Verfahren nach Anspruch 1, dadurch gekennzeichnet, daß man bei Ende der Extraktion im wesentlichen bei Extraktionstemperatur und -druck einen aliquoten Teil dieses Extrakts entnimmt, ausgehend von dem man dann dieses Signal erzeugt.

4. Verfahren nach einem der Ansprüche 1 bis 3, dadurch gekennzeichnet, daß das verwendete Lösungsmittel Kohlendioxid ist.

5. Verfahren nach einem der Ansprüche 1 bis 3, dadurch gekennzeichnet, daß das verwendete Lösungsmittel wenigstens einen Kohlenwasserstoff mit 3 bis 12 Kohlenstoffatomen umfaßt.

6. Verfahren nach einem der Ansprüche 2 bis 5, dadurch gekennzeichnet, daß man dieses Signal ausgehend von einer Chromatographieanalyse erzeugt.

7. Verfahren nach Anspruch 6, dadurch gekennzeichnet, daß man dieses Signal aufgrund einer Chromatographieanalyse in gasförmiger Phase erzeugt.

8. Verfahren nach einem der Ansprüche 1 bis 7, dadurch gekennzeichnet, daß man die Extraktionsstufe bei einer Temperatur zwischen der kritischen Temperatur des Lösungsmittels und einer Temperatur vornimmt, die um etwa 100°C über dieser kritischen Temperatur liegt.

9. Vorrichtung zur Durchführung des Verfahrens nach Anspruch 1, dadurch gekennzeichnet, daß sie in Kombination umfaßt:
- Mittel (10, 19), um in geschlossenem Kreislauf (20) in der einen oder anderen Richtung ein Gemisch in Zirkulation zu versetzen, welches wenigstens ein Lösungsmittel bei einem Druck und einer Temperatur umfaßt, die wenigstens gleich der kritischen Temperatur und dem kritischen Druck dieses Lösungsmittels sind,
- ein Extraktionsorgan (5), das diese Probe enthält und in diesem Kreis angeordnet ist, wobei dieses Organ eine Extraktionslösung erzeugt, die wenigstens ein Molekül dieses Extrakts enthält und
- wenigstens ein Meßgerät (15), das ein für diesen Parameter repräsentatives Signal ausgehend von diesem Molekül dieses Extrakts erzeugt, wobei dieser Parameter im wesentlichen bei diesen Extraktionstemperaturen und Drücken gemessen wird, wobei die Vorrichtung mit diesem Extraktionsorgan (5) und diesen Mitteln, um in Zirkulation zu versetzen (10), verbunden ist.

10. Vorrichtung nach Anspruch 9, dadurch gekennzeichnet, daß sie im übrigen wenigstens ein Organ (13) umfaßt, das bei diesen Extraktionstemperaturen und -drücken einen aliquoten Anteil dieses Extrakts entnimmt und zwischen dem Extraktionsorgan und dem Gerät zur Erzeugung und Messung dieses Signals zwischengeschaltet ist.

11. Vorrichtung nach einem der Ansprüche 9 oder 10, dadurch gekennzeichnet, daß die Mittel, die die Zirkulation hervorrufen, ein Pumporgan (10) unter Druck, das mit dem Extraktionsorgan (15) verbunden ist, sowie Heizmittel (19) für dieses Extraktionsorgan (5) dieses Entnahmeorgans (13) und dieses Kreises (20) umfassen.

12. Vorrichtung nach einem der Ansprüche 9 bis 11, dadurch gekennzeichnet, daß das dieses Signal erzeugende Meßgerät (15) einen Chromatographen, der wenigstens einen Detektor (17) aufweist, umfaßt.

13. Vorrichtung nach Anspruch 12, dadurch gekennzeichnet, daß der Chromatograph ein Chromatograph in gasförmiger Phase ist und daß der Detektor gewählt ist aus der Gruppe, die umfaßt: Flammenionisationsdetektoren, Katharometer, Flammenfotometrie-, Fotoionisations- und Infrarotlichtdetektoren sowie Massenspektrometer.

14. Anwendung des Verfahrens nach einem der Ansprüche 1 bis 8 auf die Extraktion von in Sedimentgesteinen enthaltenen Kohlenwasserstoffen.
